(19) Europäisches Patentamt

European Patent Office

Office européen des brevets

(11) Publication number : **0 635 275 A1**

# (12) EUROPEAN PATENT APPLICATION

(21) Application number : **94304615.1**

(22) Date of filing : **24.06.94**

(51) Int. Cl.[6] : **A61L 15/22**

(30) Priority : **29.06.93 GB 9313352**

(43) Date of publication of application :
**25.01.95 Bulletin 95/04**

(84) Designated Contracting States :
**DE DK FR GB**

(71) Applicant : **Du Pont Canada Inc.**
**Box 2200,**
**Streetsville Postal Station**
**Mississauga, Ontario L5M 2H3 (CA)**

(72) Inventor : **Robson, Russell**
**931 Heaton Road**
**Kingston, Ontario K7P 1Y7 (CA)**

(74) Representative : **Harrison, David Christopher**
**et al**
**MEWBURN ELLIS**
**York House**
**23 Kingsway**
**London WC2B 6HP (GB)**

(54) **Absorbent structure of blends of cellulosic and polyethylene pulp.**

(57) An absorbent structure comprising a low density cellulosic core formed from a blend of 70-97% by weight of cellulosic fibres and 3-30% by weight of polyethylene pulp, based on the total weight of the cellulosic fibres and polyethylene pulp, is disclosed. The polyethylene pulp is formed from polyethylene having a density in the range of 0.930-0.965 g/cm$^3$ and a melt index of less than 8 dg/min. The polyethylene pulp has monocomponent fibres and the core has a matrix of bonded polyethylene pulp. The blend may contain super absorbent polymer. Absorbent structures of improved properties are obtained e.g. for use in diapers and other absorbent structures.

EP 0 635 275 A1

The present invention relates to an absorbent web structure which has both mechanical strength properties and absorbing properties. More specifically, the invention relates to an absorbent material comprised of a blend of polyolefin pulp and cellulosic pulp that has been thermally bonded together to form an absorbent structure where the fibres of the polyolefin pulp are only partially melted and form a matrix of fibres, with improved properties at equivalent polyethylene pulp contents or equivalent properties at reduced polyethylene pulp contents.

Polyethylene pulp is formed by flash spinning of fibres from solutions of polyethylene in solvent at temperatures above the melting point of the polymer. For instance, the fibres are normally in the form of discontinuous fibres with a length of 1-10 mm which are refined to a length of 0.8-1.20 mm to form a polyethylene pulp. Such pulp has been used as a binder fibre in low density cellulosic webs, for example as absorbent pads in disposable products e.g. diapers and feminine hygiene products.

With the addition of polyolefin pulps to cellulosic pulps to form absorbent cores, improved performance, especially increased web integrity, may be obtained by thermally bonding the synthetic pulp to the cellulosic pulp. This has been described as involving melting of the synthetic pulp to form so-called "solder joints" of collapsed fibrils in the cellulosic matrix e.g. see "Thermal Bonding of Absorbent Cores - Do's and Don'ts" by Steven C. Apostolico, IMPACT 89, Miller Freeman Publications, 500 Howard St., San Francisco CA 94105 USA. The creation of the solder joints by melting and flowing the polyethylene to provide junctions with the cellulose fibres was stated therein to give the bonded core its strength. The strength of the core was defined by the bonding index, K, which was defined as follows:

$$ K = \frac{\text{force to break in grams}}{(\text{width})(\text{density})(\text{basis wt.})(\% \text{ cellulose})(\% \text{ PE})} $$

where PE = polyethylene.

The paper reported K values of 50-60 cm⁴/g.

In an alternative system, bicomponent binders have been used, with the sheath component acting as a binder on heating to facilitate bonding of the bicomponent fibres together to form a matrix, like a hot melt adhesive, and the core component remaining intact as a fibre. Such a system is described by B. Marcher in "Tailormade polypropylene and bicomponent fibres for the nonwovens industry" Tappi, December 1991 p 103-107. The retention of the core component in the form of a fibre results in an absorbent web with high tensile strength. The K values reported were 90 cm⁴/g.

It has now been found that an improved absorbent structure can be formed using polyethylene pulp bonded together in low density cellulosic webs without complete melting and flowing of the polyethylene fibrils and without the resulting loss of fibre integrity, the fibre of the polyethylene pulp being a monocomponent fibre. As reported hereinafter, K values in excess of 100 cm⁴/g may be obtained.

Accordingly, the present invention provides an absorbent structure comprising a low density cellulosic core formed from a blend of 80-97% by weight of cellulosic fibres and 3-20% by weight of polyethylene pulp, based on the total weight of the cellulosic fibres and polyethylene pulp, said polyethylene pulp being formed from polyethylene having a density in the range of 0.930-0.965 g/cm³ and a melt index of less than 8 dg/min, said polyethylene pulp having monocomponent fibres and said core having a matrix of bonded polyethylene pulp.

In a preferred embodiment of the absorbent structure of the present invention, the blend of cellulosic fibres and polyethylene pulp additionally contains super absorbent polymer.

In a further embodiment, the blend contains 80-90% by weight of cellulosic fibres and 10-20% by weight of polyethylene pulp.

In a still further embodiment, the polyethylene has a melt index of less than 2 dg/min.

In another embodiment, the polyethylene pulp is obtained by flash spinning discontinuous fibres of said polyethylene, preferably followed by refining said fibres to a length in the range of 0.8-1.20 mm.

In yet another embodiment, the polyethylene has a density of greater than 0.940 g/cm³, and especially greater than 0.950 g/cm³.

In still another embodiment, the structure has been formed by heating the blend, preferably a compacted blend, by passing hot fluid, especially air, therethrough.

The present invention is directed to an absorbent structure formed from cellulosic fibres and polyethylene pulp, sometimes referred to as a thermally bonded low density cellulosic core. The cellulosic fibres are typically wood pulp fibres and are of the type known to be used in absorbent cores e.g. in diapers, feminine hygiene products, disposable wipes and incontinence products. The grades of the wood pulp and the methods of production of the pulp are known to those skilled in the art, for example see, "Pulp", Kirk-Othmer, Encyclopedia of Chemical Technology, 3rd ed. (New York: 1982) vol 19, pp 379419.

The polyethylene pulp is formed from fibres manufactured in a flash spinning process, particular examples of which are described in Canadian patent applications No.s 2 102 568 and 2 102 578, both of S. Cloutier, L.M. Morin and V.G. Zboril. In such a process of manufacture, polyethylene is dissolved in an organic solvent and flash spun into discontinuous fibres. The polyethylene may be a high molecular weight homopolymer of ethylene or copolymer of ethylene and at least one $C_4$-$C_{10}$ hydrocarbon alpha-olefin e.g. butene-1, hexene-1 and/or octene-1. A wide variety of such polymers, including by type of monomer(s) used, molecular weight, molecular weight distribution and other properties, are commercially available. The density may be in the range of 0.930-0.965 g/cm$^3$. The melt index of the polyethylene is less than 2 dg/min i.e. in the range of from so-called "no-flow" e.g. less than about 0.01 dg/min, to 2 dg/min, especially in the range of 0.30 to 2.0 dg/min and more preferably 0.30 to 1.0 dg/min; melt index is measured by the method of ASTM D-1238 (condition E). The polyolefin may contain additives e.g. antioxidants, wetting agents, surfactants and other additives known for use in polyethylene, especially polyethylene used in the form of fibres. Such fibres are examples of monocomponent fibres i.e. the entire fibre is formed from the same polymer composition.

In a flash-spinning process, a solution of polyethylene in organic solvent is fed through a feed section to a spinneret exit to form plexifilamentary film-fibril strands, the strands being formed as the polymer solution passes from the spinneret exit. The solution is at a pressure that is at least the autogenous pressure and at a temperature sufficient to maintain the polyethylene in solution. The temperature and pressure used, and the composition of the solution especially the percent of polymer in the solution, affect the properties of the film-fibril strands obtained on spinning and consequently the fibrous material subsequently formed in the process. For instance, the temperature, pressure and solution composition may be selected so that highly oriented'fibres are obtained, such fibres being preferred. In embodiments, a mixture of steam and water is contacted with the solution passing from the spinneret exit substantially simultaneously with the passage of the solution from the spinneret exit. In a preferred embodiment, as described in the aforementioned patent applications of S. Cloutier, L.M. Morin and V.G. Zboril, the ratio of steam to water is in the range of 20:80 to 80:20 on a weight basis, especially in the range of 35:65 to 65:35. The temperature of the inert fluid is 2-40°C lower than the melting point of the polymer. The fibre may be converted to a polyethylene pulp by feeding the fibres to a refining process that reduces the length of the fibres to less than about 2 mm and with an average length in the range of about 0.80-1.20 mm as well as opening up the fibre structure. The fibres are fed to the refiner in the form of a slurry e.g. about 2% by weight, with polyvinyl alcohol added as surfactant; other surfactants may be used in combination with or instead of polyvinyl alcohol. The fibres must be of a length of not more than about 10 mm, preferably with an average length of about 6 mm in order to produce an acceptable slurry. The refining process may be carried out in a pulp and paper-type refiner. Suitable refiners include single disk, twin-flow and conical refiners.

Polyethylene pulp is polyethylene fibre having a very short length. As used herein, the pulp has an average length of less than about 2 mm, especially in the range of 0.8-1.2 mm, and preferably about 1 mm.

The blend of cellulosic fibres and polyethylene pulp is formed from 80-97% by weight of cellulosic fibres and 3-20% by weight of polyethylene pulp, based on the total weight of the cellulosic fibres and polyethylene pulp. In preferred embodiments, the blend contains 80-90% by weight of cellulosic fibres and 10-20% by weight of polyethylene pulp. The blend of cellulosic fibres and polyethylene pulp can be formed by any number of physical blending techniques. Methods can include wet forming techniques as used in the paper making industry and dry blending techniques as used in air laid products. After blending, the cellulosic fibres and synthetic pulp are formed into a low density pad using conventional air forming techniques such as defibration with a hammermill followed by air forming.

In order to thermally bond the blend of cellulosic fibres and polyethylene pulp, the blend in the form of sheet is laid on an open mesh belt and fed through bonding apparatus. The bonding apparatus is typically a hot-air bonder in which a stream of hot air is drawn through the sheet as the sheet is passed through the bonder. In a commercial process, the residence time in the bonder is typically in the range of 2 to 30 seconds. However, the residence time in the bonder and the temperature of the hot air are controlled so that the polyethylene of the pulp becomes partially molten, and forms a matrix of bonded polyethylene. If the combination of time and temperature is inadequate, the polyethylene is not bonded together to form a matrix. If the combination of time and temperature is excessive, a matrix of bonded polyethylene is formed but the matrix lacks strength, especially tensile strength, because the polyethylene has become fully molten with a resultant loss of orientation and other physical properties.

The bonded core that passes from the bonder is then cooled, after which it may be used as such, or cut or trimmed into convenient shapes and sizes for use in another process.

The degree of formation of the matrix may be tested by digestion of the cellulosic fraction of the core formed in the process. This may be accomplished by following the method of ASTM D629, for quantitative analysis of textiles. A piece of the bonded core is placed in 70% sulphuric acid to dissolve the cellulose fraction and leave behind the synthetic pulp portion. The matrix structure is identifiable by the remaining skeletal structure of polyethylene fibres which remain together in a cohesive unit. If the matrix structure is not formed the polyethylene fibres will remain unattached to each other and resemble fine particles dispersed through the sulphuric acid.

Strength tests as described below may also be used to assess whether matrix bonding has been achieved.

In a preferred embodiment of the process, the blend is heated to a temperature in the range of ±5°C of the melting point of the polyethylene, with the time period being selected so as to partially melt the polyethylene. It will be understood that the period of time at any selected temperature will depend on other factors in the process, including the rate of air flow through the web, the degree of compaction of the web and the amount of polyethylene pulp in the blend.

The compaction of the blend of cellulosic fibres and polyethylene pulp may be measured and expressed as a density. It is preferred that the compacted blend have a density in the range of 0.03 to 0.20 g/cm$^3$, especially in the range of 0.05 to 0.12 g/cm$^3$ and particularly in the range of 0.07 to 0.10 g/cm$^3$.

In a preferred embodiment of the absorbent structure of the invention, the polyethylene has a density of greater than 0.950 g/cm$^3$ and a melt index of less than 1 dg/min, and the compacted blend has a density of 0.05-0.12 g/cm$^3$.

The strength of the structure of the core is primarily from the matrix of interconnected bonded polyethylene pulp fibres as opposed to mechanical bonding of cellulose fibres to cellulose fibres. This results in the formation of an absorbent structure that has a greater dry strength integrity and especially a higher wet strength integrity.

In preferred embodiments, the absorbent core contains superabsorbent polymer (SAP), for example up to 60% by weight of SAP and especially 20-40% by weight of SAP, based on the total amount of cellulosic fibres, polyethylene pulp and SAP.

The absorbent structures of the present invention may be used in a variety of end-uses, an important example of which is in absorbent cores e.g. in diapers, feminine hygiene products, disposable wipes and incontinence products.

The present invention is illustrated by the following examples.

## Example I

Sheets were made by mixing cellulose and polyethylene pulp together in the form of a slurry, using a Tappi Pulp Disintegrator. The sheet was formed in a 20 x 20 cm handsheet former, and then dried in a laboratory forced air oven; the handsheets had a weight of about 25g. The handsheets were converted into fluff pads using a Waring blender with each pad 7.5 cm in diameter and weighing 1.14 g.

The absorbent pads contained 85 % of cellulose and 15 % of Plexafil™ DP700 polyethylene pulp, available from Du Pont Canada Inc. As tested, the pads had been compacted to a density of 0.06 g/cm$^3$.

The absorbent pads were thermally bonded by a through-air bonding technique using hot air from a laboratory forced air oven and a vacuum system to pull the hot air through the pads. The air velocity was approximately 120 metres/min. The temperature of the air was controlled in the oven to within ±1°C and was monitored using a thermocouple placed at the exit vent from the oven, just below the absorbent pad. The pad was held in place using a wire mesh screen with tissue being used to prevent loss of fibres. The pads were maintained at a consistent thickness, and hence a consistent density, using metal ring calipers.

The absorbent pads were tested for tensile strength using a procedure similar to ASTM D5035 "Standard Test Method for Breaking Force and Elongation of Textile Fabrics". The thermally bonded pads were cut into strips measuring 2.54 cm in width and 7.5 cm in length, and tested for tensile strength on an Instron® tester. A jaw separation of 2.54 cm was used, due to the small sample size, and the cross-head speed was 2.54 cm/min.

In a series of runs, absorbent pads were thermally bonded over a range of temperatures, using a bonding time of 10 seconds, and the tensile strength of the resultant thermally bonded pads was measured. Further details and the results obtained are given in Table I; in all cases the results given are the average of at least 4 duplicate samples.

TABLE I

| Temperature (°C) | Tensile Strength (g/2.5 cm) | Normalized Tensile Strength |
|---|---|---|
| 132 | 119 | 0.412 |
| 134 | 180 | 0.622 |
| 136 | 248 | 0.860 |
| 138 | 289 | 1.000 |
| 140 | 265 | 0.918 |
| 142 | 232 | 0.803 |
| 144 | 196 | 0.680 |

Note: Normalized tensile strength is the ratio of the tensile strength obtained to the maximum tensile strength obtained i.e. at 138°C.

This example shows that the tensile strength is very sensitive to the temperature of the hot air used in the thermal bonding process. The maximum tensile strength was obtained when the temperature of the air was the near the melting point of the polyethylene as measured using a differential scanning calorimeter. The tensile strength rapidly decreased as the air temperature deviated above or below the melting point. The tensile strength at lower temperatures reflects insufficient bonding together of the fibres below the melting point. The tensile strength at higher temperatures reflects loss of physical properties, especially orientation, of the fibres.

Example II

The procedure of Example I was repeated, using different blend compositions, bonding times, bonding temperatures and blend densities. The polyethylene was the same as that used in Example I.

Further details and the results obtained are given in Table II.

TABLE II

| Synthetic Pulp (%) | Bonding Time (sec) | Bonding Temp. (°C) | Blend Density (g/cm³) | Tensile Strength (g/2.5 cm) |
|---|---|---|---|---|
| 15 | 5 | 138 | 0.045 | 172 |
| 15 | 5 | 138 | 0.070 | 210 |
| 15 | 5 | 150 | 0.045 | 121 |
| 15 | 5 | 150 | 0.070 | 170 |
| 15 | 20 | 138 | 0.045 | 186 |
| 15 | 20 | 138 | 0.070 | 363 |
| 15 | 20 | 150 | 0.045 | 138 |
| 15 | 20 | 150 | 0.070 | 250 |
| 20 | 5 | 138 | 0.045 | 239 |
| 20 | 5 | 138 | 0.070 | 311 |
| 20 | 5 | 150 | 0.045 | 152 |
| 20 | 5 | 150 | 0.070 | 224 |
| 20 | 20 | 138 | 0.045 | 283 |
| 20 | 20 | 138 | 0.070 | 344 |
| 20 | 20 | 150 | 0.045 | 233 |
| 20 | 20 | 150 | 0.070 | 327 |

The results show that all four of the variables studied have effects on the tensile strength of the thermally bonded absorbent pad. Increases in the loading i.e. polyethylene pulp content, bonding time and pad density all resulted in increases in the tensile strength of the thermally bonded pads. Thus, bonding with higher contents of polyethylene pulp for longer bonding times and at higher densities results in improved tensile strength. The increase in bonding temperature resulted in a decrease in tensile strength, as would be expected in the light of the results in Example I.

Example III

This example illustrates the use of larger scale equipment to form the improved absorbent web structure. For this example, a blendsheet commercially available from Weyerhauser under the trade mark Kittyhawk II, which is a blend of Plexafil™ polyethylene pulp with cellulose fibre, was used. The absorbent web was formed on a 60 cm. Danweb pilot line. A series of runs were carried out using constant operating conditions except for the through air bonder temperature which was varied from 132°C to 170°C. The Kittyhawk II blendsheet was fluffed using a hammermill and was air formed using two Danweb designed forming heads. The web was formed on a tissue and thermally bonded in an electrically heated through air oven controlled to within 0.1°C as measured at a single point at the centre of the oven approximately 25 cm. above the web. The air was pulled downward through the web. The web had a residence time of 12 seconds in the oven. The web was controlled to a basis weight of approximately 130 grams per square metre and the density was set at approximately 0.08 g/cm³. The web was compacted both before and after thermal bonding to control the web density.

Details of the runs are given in Table III. The results in all cases are the average of at least 4 duplicate samples.

TABLE III

| Oven Temp. (°C) | Dry Tensile Strength (g/2.5 cm) | Wet Tensile Strength (g/2.5 cm) | Dry K Value | Wet K Value | Matrix Structure |
|---|---|---|---|---|---|
| 132 | 283 | 165 | 66.7 | 39.0 | Partial |
| 134 | 334 | 197 | 107.3 | 63.4 | Complete |
| 135 | 360 | 247 | 102.5 | 70.4 | Complete |
| 136 | 344 | 222 | 106.4 | 68.7 | Complete |
| 138 | 328 | 196 | 91.2 | 54.5 | Complete |
| 141 | 248 | 93 | 74.3 | 27.9 | Complete |
| 144 | 210 | 76 | 54.6 | 19.9 | Partial |
| 147 | 177 | 55 | 46.8 | 14.7 | Partial |
| 150 | 176 | 51 | 41.3 | 12.1 | None |
| 170 | 163 | 33 | 40.3 | 8.1 | None |

These results, as in Example I, show the sensitivity of the tensile strength to the temperature of the hot air used in the thermal bonding process. In addition, as in Example I, the maximum tensile strength was obtained near the melting point of the polyethylene pulp.

This Example shows the ability to achieve the improved absorbent structure on larger scale equipment as compared to Example I.

Example IV

The procedure of Example III was repeated using different bonding times, bonding temperatures and web densities. The bonding time was varied by increasing or decreasing the line speed. The web densities were varied by varying the degree of pre- and post-compaction through the oven. The raw material used for these runs was the same as Example III i.e. Kittyhawk II pulp with a polyethylene pulp content of 20%.

Further details and the results obtained are given in Table IV.

Table IV

| Oven Temp. (°C) | Bond Time (sec) | Web Density (g/cm³) | Dry Tensile (g/2.5cm) | Wet Tensile (g/2.5cm) | Dry K Value | Wet K Value (g/g) | GATS* Capacity | Matrix |
|---|---|---|---|---|---|---|---|---|
| 136 | 6 | 0.051 | 145 | 97 | 58.1 | 38.8 | 15.8 | yes |
| 136 | 6 | 0.080 | 301 | 222 | 75.8 | 55.9 | 11.3 | yes |
| 136 | 20 | 0.089 | 456 | 266 | 110.5 | 64.3 | 10.2 | yes |
| 136 | 20 | 0.048 | 163 | 114 | 80.4 | 56.4 | 15.9 | yes |
| 150 | 20 | 0.049 | 79 | 23 | 35.6 | 10.2 | 15.9 | yes |
| 150 | 20 | 0.074 | 162 | 40 | 48.0 | 11.8 | 11.9 | no |
| 150 | 6 | 0.068 | 132 | 23 | 39.4 | 6.9 | 12.1 | no |
| 150 | 6 | 0.048 | 67 | 18 | 28.3 | 7.5 | 15.2 | no |

*GATS = Gravimetric Absorbency Testing System is a measure of the rate of absorption of fluids by absorbent materials. A general description of the system can be found in "Absorbency Textile Science and Technology, Vol. 7, Ed. Pronoy K. Chatterijce, Published by Elsevier. The procedure used was according to the manual for the M/K Systems, Inc. Second Generation Gravimetrics Absorbency Testing System dated May 1986, model M/K 201. A porous test plate was used and the absorption fluid was a 0.9% saline solution.

This Example shows that a lower bonding temperature resulted in improved dry and wet tensile strength over a range of bonding times and pad densities. In addition, this example shows that by bonding and forming a matrix structure, a web can be made at a lower density (0.05 g/cm³) that has both a higher wet strength and a higher dry strength than a much higher density web (0.08 g/cm³). This is an important advantage since a lower density web has a higher absorptive capacity. A lower density web also has a lower web integrity. However, by forming a matrix structure, a web may be made that combines a low density for improved absorptive properties and a high strength for improved mechanical properties.

Example V

This example illustrates the ability to reduce the synthetic pulp content in the absorbent core by forming the matrix structure, while retaining web properties of an absorbent core obtained using the traditional solder bond point.

The absorbent webs were formed using the procedures of Examples III and IV.

The absorbent webs of this example contained super absorbent polymers (SAP) which are widely used to improve. the liquid absorptive properties of the web. The absorbent webs were controlled to a basis weight of approximately 209 g/m². The SAP was dosed through the forming heads of the air lay equipment used to form a homogeneous blend of cellulose pulp, synthetic pulp and SAP; the SAP used was obtained from Atochem under the trade designation 10SHP.

Further details and the results obtained are given in Table V.

TABLE V

| Synthetic Pulp (%) | SAP (%) | Density (g/cm³) Length (m) | Dry Break Length (m) | Wet Break | GATS (g/g) | Retention (g/g) | Matrix Sructure |
|---|---|---|---|---|---|---|---|
| 20 | 40 | 0.069 | 4.9 | 2.4 | 19.2 | 14.9 | no |
| 20 | 40 | 0.110 | 16.7 | 2.5 | 15.6 | 13.3 | no |
| 15 | 40 | 0.086 | 16.2 | 8.8 | 18.5 | 15.5 | yes |

Note:
(1) The amount of synthetic pulp (Plexafil) is based on the web without SAP;
(2) Matrix formation was determined by digestion of a sample of the web;
(3) GATS = gravimetric absorbency testing system, performed without load;
(4) Breaking length = tensile strength (g/m of width)/basis weight (g/m²);
(5) Retention = fluid retained in web after applying a 1100g weight of the fluid used in the GATS test.

From Table V, it can be seen that the matrix web has superior dry strength performance at 15% synthetic pulp content, compared with the web with 20% synthetic pulp content that does not form a matrix structure. To achieve equivalent dry strength at 20% pulp content requires higher densities, which adversely affects the liquid absorptive capacity. The web strength of the 15% pulp sample, with matrix bonding, is much higher than the wet strength of the samples without matrix bonding, showing the importance of the interconnected matrix structure compared with the discrete bond points. Thus, this example shows that it is possible to reduce the content of the synthetic pulp and also use a lower density, and still form a web with superior web integrity, and with higher absorptive capacity and retention.

Example VI

The procedure of Example V was repeated, varying the amount of Plexafil DP700 synthetic pulp in the final product by addition of cellulose pulp using a hammermill. The bonding temperature was maintained at 136°C, which was shown in Example III to form a matrix structure. The densities of the webs were also varied, using the procedure of Example IV.

Further details and the results obtained are given in Table VI. A matrix structure was formed in all the samples reported in Table VI. The SAP used was the same as that used in Example V.

TABLE VI

| Bonding Time (sec) | Basis Weight (g) | Density (g/cm$^3$) | SAP (%) | Synthetic Pulp (%) | Dry Tensile Strength (g/2.5cm) | Dry Breaking Length (m) |
|---|---|---|---|---|---|---|
| 6 | 190.3 | 0.073 | 30 | 15 | 103.6 | 21.4 |
| 6 | 209.4 | 0.089 | 30 | 15 | 154.1 | 28.9 |
| 6 | 203.2 | 0.100 | 30 | 15 | 203.7 | 39.4 |
| 6 | 178.0 | 0.093 | 40 | 15 | 107.7 | 23.8 |
| 6 | 177.3 | 0.074 | 20 | 15 | 124.1 | 27.5 |
| 6 | 184.5 | 0.066 | 30 | 10 | 45.0 | 9.6 |
| 6 | 208.7 | 0.078 | 40 | 10 | 52.3 | 9.9 |
| 6 | 204.9 | 0.107 | 40 | 10 | 70.5 | 13.5 |
| 10 | 246.6 | 0.092 | 20 | 10 | 149.1 | 23.8 |
| 10 | 255.0 | 0.118 | 20 | 10 | 200.5 | 30.9 |
| 6 | 216.8 | 0.068 | 30 | 15 | 139.1 | 25.2 |
| 6 | 180.6 | 0.068 | 30 | 15 | 105.9 | 23.1 |
| 6 | 216.2 | 0.078 | 30 | 15 | 114.6 | 20.8 |
| 6 | 240.1 | 0.105 | 20 | 20 | 451.0 | 73.9 |
| 6 | 228.5 | 0.113 | 20 | 20 | 498.7 | 85.8 |
| 6 | 207.8 | 0.109 | 40 | 20 | 261.8 | 49.5 |
| 6 | 208.1 | 0.086 | 40 | 20 | 181.4 | 34.3 |
| 6 | 220.1 | 0.072 | 30 | 20 | 182.7 | 32.7 |
| 6 | 205.2 | 0.077 | 30 | 15 | 114.6 | 22.0 |

Note:    (1)    The amount of synthetic pulp was based on the web without SAP;

(2)    Matrix formation was determined by digestion of the web

This example shows that matrix structures may be obtained with cores containing SAP over a range of densities, SAP loadings and loadings of synthetic pulp.

Example VII

Blends of 20% polyethylene pulp with 80% NB316 Weyerhaeuser pulp were formed into handsheets and then into absorbent pads using the modified Waring blender. The pads weighed approximately 130 g/m$^2$ and had a density of 0.08 g/cm$^3$. The resultant pads were through air bonded using a convection oven, being bonded for 10 seconds at a constant temperature. Previous experience using similar blends in the convection oven had indicated that the highest tensile strength was obtained at about 130°C, and thus temperatures used in this example were 130°C and 150°C.

The samples were measured for tensile strength under both wet and dry conditions as well as being digested in sulphuric acid to see if a matrix structure was formed.

The results of the tensile strength measurements are shown in Table VII below.

The results of digestion in sulphuric acid were as follows: (i) all pulps formed a matrix structure when the bonding temperature was 130°C; (ii) with bonding temperatures of 150°C, polyethylene pulp identified as A, B, C and E in Table VII formed a point bond structure i.e. a matrix was not formed, polyethylene pulp F retained a matrix structure, and polyethylene pulps G and H showed partial matrix structures.

Handsheet zero span, referred to in Table VII as zero span, is a measure of fibre strength. It is obtained using the procedures of TAPPI T205 om and a Pulmac Troubleshooter, using the method suggested by the manufacturer.

10

Table VII

| Synthetic Pulp | Melt Index | Zero Span | Bonding Temp = 130°C | | Bonding Temp = 150°C | |
|---|---|---|---|---|---|---|
| | | | Dry Tensile | Wet Tensile | Dry Tensile | Wet Tensile |
| A | 0.4 | 13.8 | 0.704 | 0.221 | 0.562 | 0.087 |
| B | 0.43 | 17.2 | 0.707 | 0.245 | 0.416 | 0.070 |
| C | 0.97 | 22.2 | 0.647 | 0.327 | 0.414 | 0.085 |
| F | 1.0 | 18.4 | 0.755 | 0.398 | 0.780 | 0.150 |
| F | 1.0 | 18.7 | 0.987 | 0.437 | 0.872 | 0.157 |
| D | 5.0 | 9.9 | 0.660 | 0.133 | 0.484 | 0.102 |
| G | 7.3 | 4.5 | 1.237 | 0.267 | 1.006 | 0.130 |
| E | 9.0 | 5.9 | 0.357 | 0.116 | 0.451 | 0.108 |

Note:     The polyethylene (PE) pulps were as follows:

A =   Pulp formed from Sclair® 99C, an ethylene homopolymer having a density of 0.960 g/cm$^3$ and a melt index of 0.40 dg/min, with an average fibre length of 0.97 mm;

B =   Pulp formed from Sclair® 58E, a copolymer of ethylene and butene-1 having a density of 0.9555 g/cm$^3$ and a melt index of 0.43 dg/min, with an average fibre length of 1.03 mm;

C =   Plexafil DP700 pulp, formed from an ethylene homopolymer having a density of 0.960 g/cm$^3$ and a melt index of 0.97 dg/min, with an average fibre length of 0.90 mm;

D =   Pulp formed from Sclair® 2907, an ethylene homopolymer having a density of 0.960 g/cm$^3$ and a melt index of 5.0 dg/min, with an average fibre length of 0.90 mm;

E =   polyethylene pulp obtained from Mitsui

under the trade designation E400;

F = Pulp formed from Sclair® 58G, a polyethylene having a density of 0.956 g/cm³ and a melt index of 1.0 dg/min, with an average fibre length of 1.13 mm;

G = Pulp formed from Sclair® 2908, a copolymer of ethylene and butene-1 having a density of 0.960 g/cm³ and a melt index of 7.3 dg/min, with an average fibre length of 0.98 mm;

For most of the pulps, there was a decrease in the dry tensile strength at the higher bonding temperature, which is most readily apparent in the wet tensile measurements. The best correlation was found to be between the tensile strength measurements and the pulp fibre strength as measured using handsheet zero span.

At low bonding temperatures, it would seem that the tensile strength of the fibres is important in addition to formation of a matrix. Thus, high strength pulps such as polyethylene pulps C and F formed the strongest structures, especially compared with the structure formed from the low strength commercial pulp. Conversely, at the higher bonding temperature the decrease in tensile strength is most dramatic for the high strength pulps. Such pulps have a high degree of orientation and correspondingly have a high shrinkage, and therefore tend to lose the matrix structure more easily. This results in loss of strength of the web. Lower strength pulps are believed to be less affected by the higher temperature but this is not useful to the properties of the absorbent structure because the fibres are weak and easily pulled apart.

## Claims

1. An absorbent structure comprising a low density cellulosic core formed from a blend of 80-97% by weight of cellulosic fibres and 3-20% by weight of polyethylene pulp, based on the total weight of the cellulosic fibres and polyethylene pulp, said polyethylene pulp being formed from polyethylene having a density in the range of 0.930-0.965 g/cm³ and a melt index of less than 8 dg/min, said polyethylene pulp having monocomponent fibres and said core having a matrix of bonded polyethylene pulp.

2. The absorbent structure of Claim 1 in which the blend of cellulosic fibres and polyethylene pulp additionally contains super absorbent polymer.

3. The absorbent structure of claim 1 or claim 2 in which the blend contains 80-90% by weight of cellulosic fibres and 10-20% by weight of polyethylene pulp.

4. The absorbent structure of any preceding claim in which the polyethylene pulp is obtained by flash spinning discontinuous fibres of said polyethylene.

5. The absorbent structure of Claim 4 in which the polyethylene pulp has been refined to a fibres length in the range of 0.8-1.20 mm.

6. The absorbent structure of any preceding claim in which the polyethylene has a density of greater than 0.940 g/cm³.

7. The absorbent structure of Claim 6 in which the density is greater than 0.950 g/cm³.

8. The absorbent structure of any one of Claims 1-7 in which the blend is compacted to a density in the range of 0.03 to 0.20 g/cm³.

9. The absorbent structure of Claim 8 in which the compacted density is in the range of 0.05 to 0.12 g/cm³.

10. The absorbent structure of Claim 9 in which the compacted density is in the range of 0.07 to 0.10 g/cm$^3$.

11. The absorbent structure of any one of Claims 1-10 in which the polyethylene has a melt index of less than 2 dg/min.

12. A method of making an absorbent structure which includes thermally bonding a blend of fibres and pulp as defined in any preceding claim , without complete melting of the polyethylene fibres.

European Patent
Office

**EUROPEAN SEARCH REPORT**

Application Number

EP 94 30 4615

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 391 076 (HERCULES INC.) <br> * claims; examples * <br> --- | 1,4 | A61L15/22 |
| X | EP-A-0 317 058 (HERCULES INC.) <br> * claims * <br> --- | 1 | |
| Y | EP-A-0 462 620 (E.I. DU PONT DE NEMOURS AND COMPANY.) <br> * claims * <br> --- | 1-12 | |
| D,P, Y | EP-A-0 598 536 (DU PONT CANADA INC.) <br><br> * claims * <br> & CA-A-2 102 568 <br> --- | 1-12 | |
| D,P, Y | EP-A-0 597 658 (DU PONT CANADA INC.) <br><br> * claims * <br> & CA-A-2 102 578 <br> --- | 1-12 | |
| A | FR-A-2 628 761 (LA CELLULOSE DU PIN.) <br> * claims; examples * <br> --- | 1 | TECHNICAL FIELDS SEARCHED (Int.Cl.6) <br><br> A61L |
| A | EP-A-0 070 164 (CHICOPEE) <br> * claims * <br> ----- | 1 | |

The present search report has been drawn up for all claims

| Place of search | Date of completion of the search | Examiner |
|---|---|---|
| THE HAGUE | 4 November 1994 | ESPINOSA, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)